# EUROPÄISCHE PATENTSCHRIFT

(11) **EP 0 944 675 B1**
(45) Veröffentlichungstag und Bekanntmachung des Hinweises auf die Patenterteilung: **14.08.2002**
(21) Anmeldenummer: 97952027.7
(22) Anmeldetag: 01.12.1997
(51) Int. Cl.: C09B 61/00, A23L 1/275

(54) **STABILE, WÄSSRIGE DISPERSIONEN UND STABILE, WASSERDISPERGIERBARE TROCKENPULVER VON XANTHOPHYLLEN, DEREN HERSTELLUNG UND VERWENDUNG**
STABLE, AQUEOUS DISPERSIONS AND STABLE, WATER-DISPERSIBLE DRY XANTHOPHYLL POWDER, THEIR PRODUCTION AND USE
DISPERSIONS AQUEUSES STABLES ET POUDRE SECHE STABLE DISPERSIBLE DANS L'EAU DE XANTHOPHYLLES, LEUR PREPARATION ET LEUR UTILISATION

(30) Priorität: 12.12.1996 DE 19651681
(43) Veröffentlichungstag der Anmeldung: 29.09.1999
(73) Patentinhaber: BASF AKTIENGESELLSCHAFT, 67056 Ludwigshafen (DE)
(72) Erfinder: AUWETER, Helmut, D-67117 Limburgerhof (DE); BOHN, Heribert, D-67319 Wattenheim (DE); LÜDDECKE, Erik, D-67112 Mutterstadt (DE)
(86) Internationale Anmeldenummer: EP9706712
(87) Internationale Veröffentlichungsnummer: WO9826008

(56) Entgegenhaltungen:
- EP-A- 0 065 193
- EP-A- 0 239 949
- EP-A- 0 278 284
- EP-A- 0 410 236
- WO-A-91/06292
- WO-A-94/19411
- DE-A- 2 411 529
- DE-B- 1 211 911
- DE-B- 1 288 713
- DR. OTTO-ALBRECHT NEUMÜLLER: "Römpps Chemielexikon" 1981 , FRANCKH'SCHE VERLAGSHANDLUNG , STUTTGART, DE XP002059429 siehe Seite 919 "Dextrine" siehe Seite 1426 "Gelatine"

## Beschreibung

Die Stoffklasse der Carotinoide klassifiziert man in zwei Hauptgruppen, die Carotine und die Xanthophylle. Im Unterschied zu den Carotinen. bei denen es sich um reine Polyen-Kohlenwasserstoffe handelt. wie beispielsweise β-Carotin oder Lycopin, kommen in den xanthophyllen noch Sauerstoff-Funktionen wie Hydroxy-, Epoxyund/oder Oxogruppen vor. Typische Vertreter dieser Gruppe sind u.a. Astaxanthin, Canthaxanthin und Zeaxanthin.

Xanthophylle sind in der Natur weit verbreitet und kommen u.a. im Mais (Zeaxanthin), in grünen Bohnen (Lutein), in Paprika (Capsanthin), in Eidottern (Lutein) sowie in Krebsen und Lachsen (Astaxanthin) vor, wobei sie diesen Nahrungsmitteln ihre charakteristische Färbung verleihen.

Diese teilweise technisch herstellbaren sowie aus natürlichen Quellen isolierbaren Polyene stellen für die Lebens- und Futtermittelindustrie und für den pharmazeutischen Bereich als Ersatz für künstliche Farbstoffe wichtige Farbkörper dar.

Alle Xanthophylle sind in Wasser unlöslich, während in Fetten und Ölen eine jedoch nur geringe Löslichkeit gefunden wird. Diese begrenzte Löslichkeit sowie die hohe Oxidationsempfindlichkeit stehen einer direkten Anwendung der relativ grobkörnigen bei der Synthese erhaltenen Produkte in der Einfärbung von Lebens- und Futtermitteln entgegen, da die Substanzen in grobkristalliner Form nur schlechte Färbungsergebnisse liefern. Diese für die praktische Verwendung der Xanthophylle nachteiligen Effekte wirken sich insbesondere im wäßrigen Medium aus, da sie darin gänzlich unlöslich sind.

Nur durch gezielt hergestellte Formulierungen, in denen die Wirkstoffe in fein verteilter Form und gegebenenfalls durch Schutzkolloide oxidationsgeschützt vorliegen, lassen sich bei der direkten Einfärbung von Lebensmitteln verbesserte Farbausbeuten erzielen. Außerdem führen diese in Futtermitteln verwendeten Formulierungen zu einer höheren Bioverfügbarkeit der Xanthophylle und damit indirekt zu besseren Färbungseffekten z.B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 µm zu bringen.

Zahlreiche Methoden, u.a. beschrieben in Chimia 21, 329 (1967), WO 91/06292 sowie in WO 94/19411, bedienen sich dabei der Vermahlung von Carotinoiden mittels einer Kolloidinühle und erzielen damit Partikelgrößen von 2 bis 10 µm.

Daneben existieren eine Reihe von kombinierten Emulgier-/Sprühtrocknungsverfahren, wie sie z.B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen β-Carotinpräparaten dadurch, daß man β-Carotin in einem flüchtigen, mit Wasser mischbaren organischen Lösungsmittel bei Temperaturen zwischen 50°C und 200°C, gegebenenfalls unter erhöhtem Druck, innerhalb einer Zeit von weniger als 10 Sekunden löst. Aus der erhaltenen molekulardispersen Lösung wird das β-Carotin durch sofortiges schnelles Mischen mit einer wäßrigen Lösung eines Schutzkolloids bei Temperaturen zwischen 0°C und 50°C ausgefällt. Man erhält so ein kolloid-disperses β-Carotin-Hydrosol mit orange-gelber Farbnuance. Anschließende Sprühtrocknung der Dispersion liefert ein freifließendes Trockenpulver, das sich in Wasser unter Bildung einer klaren, gelborange gefärbten Dispersion löst.

Bei den nach EP-B-0 065 193 hergestellten nanopartikulären Wirkstoffdispersionen von Xanthophyllen sind jedoch folgende Phänomene zu beobachten.

Die wäßrigen, Xanthophyll-haltigen Wirkstoffdispersionen sind häufig, insbesondere bei der Aufkonzentration, kolloidal instabil. Durch Ausflockungen der Wirkstoffpartikel, die dabei teilweise sedimentieren, teilweise aufrahmen, ist eine weitere Überführung der Dispersion in ein Trockenpulver nicht mehr möglich.

Bei Xanthophyllen mit Carbonyl-Funktionen kann außerdem die als alleiniges Schutzkolloid eingesetzte Gelatine vernetzen, so daß ein Gel entsteht, das nicht mehr redispergierbar ist und das ebenfalls nicht weiter in ein Trockenpulver überführt werden kann.

Somit können die hohen Anforderungen an Xanthophyll-haltigen Formulierungen bezüglich Farbwirkung und Bioverfügbarkeit aufgrund der geschilderten Problematik mit dem o.g. Verfahren nicht immer erfüllt werden.

Es bestand daher die Aufgabe, ein Verfahren zur Herstellung einer stabilen, wässrigen Dispersion von Xanthophyllen vorzuschlagen. Ferner sollten stabile, pulverförmige Xanthophyll-Zubereitungen zur Verfügung gestellt werden, mit denen eine gute Farbwirkung und zudem eine hohe Bioverfügbarkeit erzielt werden können.

Diese Aufgabe wurde erfindungsgemäß gelöst mit einem Verfahren zur Herstellung einer stabilen, wäßrigen Dispersion oder eines stabilen wasserdispergierbaren Trockenpulvers von Xanthophyllen, das dadurch gekennzeichnet ist, daß man
a) eine molekulardisperse Lösung mindestens eines Xanthophylls gegebenenfalls zusammen mit einem Emulgator und/oder einem eßbaren Öl in einem mit Wasser mischbaren, organischen Lösungsmittel oder einer Mischung aus Wasser und einem mit Wasser mischbaren, organischen Lösungsmittel bei Temperatur größer 30°C herstellt,
b) diese Lösung mit einer wäßrigen Lösung eines Gemisches von Schutzkolloiden mischt,
   b₁) bei der das Gemisch mindestens eine niedermolekulare Schutzkolloid-Komponente und mindestens eine hochmolekulare Schutzkolloid-Komponente enthält, deren mittlere Molekulargewichte sich um mindestens 10000 unterscheiden,
   b₂) wobei die Lösungsmittelkomponente in die wäßrige Phase überführt wird und die hydrophobe Phase des Xanthophylls als nanodisperse Phase entsteht
c) und gegebenenfalls die gebildete Dispersion für die Herstellung eines wasserdispergierbaren Trockenpulvers von dem Lösungsmittel und dem Wasser befreit und, gegebenenfalls in Gegenwart eines Überzugsmaterials, trocknet.

Die Erfindung betrifft außerdem stabile, Xanthophyll-haltige, kaltwasserdispergierbare Trockenpulver, die sich hervorragend zum Färben von Lebens- und Futtermitteln sowie von pharmazeutischen Darreichungsformen verwenden lassen.

Die Herstellung der erfindungsgemäßen Zubereitungen erfolgt in der Regel so, daß man das/die Xanthophyll(e), gegebenenfalls zusammen mit einem Emulgator und/oder einem eßbaren Öl, in einem mit Wasser mischbaren, organischen Lösungsmittel bei Temperatu-ren, vorzugsweise zwischen 50°C und 240°C, insbesondere 100°C bis 200°C, besonders bevorzugt 140°C bis 180°C, gegebenenfalls unter Druck, löst.

Da die Einwirkung hoher Temperaturen den gewünschten hohen alltrans Isomerenanteil herabsetzen kann, löst man das/die Xanthophyll(e) möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 80 bar, vorzugsweise 30 bis 60 bar, vorteilhaft sein.

Die so erhaltene molekulardisperse Lösung versetzt man unmittelbar anschließend mit der gegebenenfalls gekühlten wäßrigen Lösung eines Schutzkolloids bevorzugt in der Weise, daß sich eine Mischungstemperatur von etwa 35°C bis 80°C einstellt.

Dabei wird die Lösungsmittelkomponente in die wäßrige Phase überführt und die hydrophobe Phase des/der Xanthophyll(e) entsteht als nanodisperse Phase.

Hinsichtlich einer näheren Verfahrens- und Apparatebeschreibung wird an dieser Stelle ausdrücklich auf EP-B-0 065 193 Bezug genommen.

Überraschenderweise wurde nun gefunden, daß man kolloidal stabile und nicht vernetzende nanopartikuläre Wirkstoffdispersionen von Xanthophyllen erhält, wenn man, anders als in den bislang beschriebenen Pormulierverfahren, bei der oben genannten Fällung eines oder mehrerer molekulardispers gelöster Xanthophylle zunächst eine wäßrige Schutzkolloidlösung mindestens einer niedermolekularen Komponente verwendet und anschließend eine weitere wäßrige Schutzkolloidlösung mindestens einer hochmolekularen Komponente zusetzt, wobei sich die mittleren Molekulargewichte der nieder- und hochmolekularen Polymere um mindestens 10000, vorzugsweise mindestens 30000 unterscheiden.

Es ist aber auch möglich, die zweistufige Fällung in einem Schritt in einem Gemisch aus mindestens einer niedermolekularen Komponente und mindestens einer hochmolekularen Komponente, deren mittlere Molekulargewichte sich um mindestens 10000, vorzugsweise mindestens 30000 unterscheiden, durchzuführen.

Als Schutzkolloide werden nieder- und hochmolekulare Komponenten von beispielsweise Gelatine, Fischgelatine, Stärke, Dextrin, Pflanzenproteine, Pektin, Gummi-Arabikum, Kasein, Kaseinat oder Mischungen davon verwendet, wobei die proteinhaitigen Schutzkolloide, insbesondere nicht gelierende, niedermolekulare Eiweißhydrolysate und höhermolekulare, gelierende Gelatine bevorzugt sind. Es können aber auch Polyvinylalkohol, Polyvinylpyrrolidon, Methylcellulose, Carboxymethylcellulose, Hydroxypropylcellulose und Alginate eingesetzt werden. Das mittlere Molekulargewicht (M_{w}) der niedermolekularen Schutzkolloid-Komponente liegt bevorzugt zwischen 10000 und 50000, insbesondere zwischen 15000 und 30000, während die hochmolekulare Komponente ein mittleres Molekulargewicht von vorzugsweise größer 60000 aufweist. Der Anteil der niedermolekularen Schutzkolloid-Komponente beträgt 5 bis 95 Gew.%, bevorzugt 20 bis 80 Gew.%, insbesondere 30 bis 60 Gew.% der gesamten Schutzkolloidmenge. Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin.

Das Verhältnis Schutzkolloid und Weichmacher zu Xanthophyll-Lösung wird im allgemeinen so gewählt, daß ein Endprodukt erhalten wird, das zwischen 0,5 und 20 Gew.-%, vorzugsweise 10 Gew.-% Xanthophyll, 10 bis 50 Gew.-% eines Schutzkolloids, 20 bis 70 Gew.-% eines Weichmachers, alle Prozentangaben bezogen auf die Trockenmasse des Pulvers, sowie gegebenenfalls geringe Mengen eines Stabilisators enthält.

Die Xanthophylle, die bei der Durchführung der Erfindung eingesetzt werden können, sind die bekannten, zugänglichen, natürlichen oder synthetischen Vertreter dieser Klasse von Verbindungen, die als farbgebende Mittel brauchbar sind, z.B. Astaxanthin, Zeaxanthin, Canthaxanthin, Capsanthin und Lutein.

Zur Erhöhung der Stabilität des Wirkstoffes gegen oxidativen Abbau ist es vorteilhaft, Stabilisatoren wie α-Tocopherol, t-Butylhydroxy-toluol, t-Butylhydroxyanisol. Ascorbinsäure oder Ethoxyquin zuzusetzen. Sie können entweder der wäßrigen oder der Lösungsmittel-Phase zugesetzt werden, vorzugsweise werden sie jedoch gemeinsam mit den Farbstoffen und gegebenenfalls zusätzlichen Emulgatoren in der Lösungsmittel-Phase gelöst. Als Emulgatoren können beispielsweise Ascorbylpalmitat, Polyglycerin-Fettsäureester, Sorbitan-Fettsäureester, Propylenglycol-Fettsäureester oder Lecithin in einer Konzentration von 0 bis 200 Gew.%, vorzugsweise 10 bis 150 Gew.%, besonders bevorzugt 20 bis 80 Gew.%, bezogen auf das/die Xanthophylle(e), verwendet werden.

Unter Umständen kann es auch vorteilhaft sein, zusätzlich in der Lösungsmittel-Phase ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen auf das/die Xanthophylle(e), zu lösen, das dann gemeinsam mit den Wirkstoffen und den genannten Zusatzstoffen beim Mischen mit der wäßrigen Phase extrem feinteilig ausgefällt wird.

Je nach Art und Menge des verwendeten Schutzkolloids erhält man eine tiefgefärbte viskose Flüssigkeit. Die Entfernung des Lösungsmittels kann beispielsweise durch Extraktion mit einem mit Wasser nicht mischbaren Lösungsmittel oder, je nach Siedepunkt in an sich bekannter Weise, z.B. durch Destillation, gegebenenfalls unter vermindertem Druck, erfolgen. In diesem Fall hat es sich als zweckmäßig und möglich erwiesen, das bei Verwendung von Isopropanol erhaltene Azeotrop ohne Wasserentfernung unmittelbar als Lösungsmittel einzusetzen. Vorzugsweise erfolgt die Lösungsmittelabtrennung jedoch gemeinsam mit der Entfernung des Wassers durch Sprühtrocknung oder Sprühgranulation.

Man erhält ein stabiles Trockenpulver, das dadurch gekennzeichnet ist, daß es von einem Schutzkolloid umhüllt ist, das mindestens eine niedermolekulare Komponente und mindestens eine hochmolekulare Komponente enthält, deren mittlere Molekulargewichte sich um mindestens 10000 unterscheiden. Bei Verwendung eines wasserlöslichen Kolloids kann dieses Trockenpulver erneut in Wasser unter Erzielung einer gleichmäßigen Feinverteilung des Wirkstoffes im Korngrößenbereich kleiner 1 µm gelöst werden. Im photochemischen Stabilitätstest erweist sich das so erhaltene Wirkstoff-Hydrosol trotz der Feinverteilung als außerordentlich stabil.

Sowohl in der wäßrigen Xanthophyll-Dispersion, als auch im daraus hergestellten Trockenpulver weist der enthaltene Wirkstoff einen, anhand von Röntgenbeugungsdiagrammen ermittelten, amorphen Anteil zwischen 70 und 100% auf. Ferner beträgt der all-trans Isomerengehalt der Xanthophylle mindestens 50%.

Die erfindungsgemäßen Zubereitungen eignen sich hervorragend als Lebens- und Futtermittelfarbstoffe. Typische Einsatzgebiete im Futtermittelbereich sind beispielsweise die Fischpigmentierung in der Aquakultur sowie die Eidotter- und Broilerhautpigmentierung in der Geflügelzucht.

In den nachfolgenden Beispielen wird die Durchführung des erfindungsgemäßen Verfahrens näher erläutert.

### Beispiel 1

In einer beheizbaren Vorlage wurden bei einer Temperatur von 30°C 40 g Astaxanthin und 15,4 g Erdnußöl in einer Lösung von 12,3 g Ethoxyquin in 288 g Isopropanol/Wasser (88/12, w/w) suspendiert. Diese Suspension wurde in einer Mischkammer bei einer Mischungstemperatur von 170°C mit 587 g Isopropanol/Wasser (88/12, w/w) bei einer Verweilzeit von 0,2 Sekunden gemischt. Nach der genannten Verweilzeit trat die entstandene molekulardisperse Astaxanthin-Lösung unmittelbar anschließend in eine weitere Mischkammer ein, in der unter einem Mischungswinkel von 90° über eine Hochdruckpumpe 11340 g einer auf pH 9 eingestellten, wäßrigen Gelatinelösung, die neben 84 g Gelatine A (100 Bloom, M_{w} = 94000), 42 g Gelita® Sol P, Deutsche Gelatine Fabrik, Eberbach (M_{w} = 21000) und 92 g Saccharose enthielt, zugemischt wurden, wobei das Astaxanthin in kolloiddisperser Form mit einer mittleren Teilchengröße von 166 nm bei einer Temperatur von 45°C ausfiel.

Anschließend wurde die Dispersion aufkonzentriert und in an sich bekannter Weise in ein freifließendes Trockenpulver mit einer mittleren Teilchengröße von 237 nm überführt. Das Trockenpulver löste sich in Wasser wiederum unter Bildung einer klaren, rot gefärbten Dispersion, wobei die Farbstärke der Re-Dispersion lediglich um ca. 10%, bezogen auf die ursprüngliche Dispersion, abnahm.

### Vergleichsbeispiel

In einer beheizbaren Vorlage wurden bei einer Temperatur von 30°C 40 g Astaxanthin und 15,4 g Brdnußöl in einer Lösung von 12,3 g Ethoxyquin in 288 g Isopropanol/Wasser (88/12, w/w) suspendiert. Diese Suspension würde in einer Mischkammer bei einer Mischungstemperatur von 170°C mit 548 g Isopropanol/Wasser (88/12, w/w) bei einer Verweilzeit von o,2 Sekunden gemischt. Nach der genannten Verweilzeit trat die entstandene molekulardisperse Astaxanthin-Lösung in eine weitere Mischkammer ein, in der unter einem Mischungswinkel von 90° über eine Hochdruckpumpe 11280 g einer auf pH 9 eingestellten, wäßrigen Gelatinelösung, die neben 126 g Gelatine A (100 Bloom, M_{w} = 94000), 91 g Saccharose enthielt, zugemischt wurden, wobei das Astaxanthin in kolloiddisperser Form mit einer mittleren Teilchengröße von 232 nm bei einer Temperatur von 45°C ausfiel.

Während des Aufkonzentrierens der Dispersion kam es zu Ausflockungen der Wirkstoffpartikel, verbunden mit einer Abnahme der Farbstärke auf 60% des Augangswertes. Mittels dynamischer Lichtstreuung wurden mittlere Teilchengrößen von 370 nm gemessen. Das nach analogem Verfahren gemäß Beispiel 1 hergestellte Trockenpulver war nur zum Teil redispergierbar.

## Patentansprüche

1. Verfahren zur Herstellung einer stabilen, wäßrigen Dispersion oder eines stabilen wasserdispergierbaren Trockenpulvers von Xanthophyllen, **dadurch gekennzeichnet, daß** man
a) eine molekulardisperse Lösung mindestens eines Xanthophylls gegebenenfalls zusammen mit einem Emulgator und/oder einem eßbaren Öl in einem mit Wasser mischbaren, organischen Lösungsmittel oder einer Mischung aus Wasser und einem mit Wasser mischbaren, organischen Lösungsmittel bei Temperaturen größer 30°C herstellt,
b) diese Lösung mit einer wäßrigen Lösung eines Gemisches von Schutzkolloiden mischt,
b₁) bei der das Gemisch mindestens ein niedermolekulares nicht gelierendes Eiweißhydrolysat und mindestens eine höhermolekulare, gelierende Gelatine enthält, deren mittlere Molekulargewichte sich um mindestens 10000 unterscheiden,
b₂) wobei die Lösungsmittelkomponente in die wäßrige Phase überführt wird und die hydrophobe Phase des Xanthophylls als nanodisperse Phase entsteht
c) und gegebenenfalls die gebildete Dispersion für die Herstellung eines wasserdispergierbaren Trockenpulvers von dem Lösungsmittel und dem Wasser befreit und, gegebenenfalls in Gegenwart eines Überzugsmaterials, trocknet.

2. Verfahren nach Anspruch 1, **dadurch gekennzeichnet, daß** der Anteil des niedermolekularen nicht gelierenden Eiweißhydrolysates 5 bis 95 Gew.% der gesamten Schutzkolloidmenge beträgt.

3. Verfahren nach den Ansprüchen 1 und 2, **dadurch gekennzeichnet, daß** die molekulardisperse Lösung des Xanthophylls in zwei Schritten mit der wäßrigen, Schutzkolloid-haltigen Phase vermischt wird, wobei die molekulardisperse Lösung des Xanthophylls zunächst nur mit dem niedermolekularen nicht gelierenden Eiweißhydrolysat und anschließend mit der höhermolekularen, gelierenden Gelatine vermischt wird, wobei sich die mittleren Molekulargewichte der nieder- und höhermolekularen Polymere um mindestens 30000 unterscheiden.

4. Stabile, wäßrige Xanthophyll-Dispersionen erhältlich nach den Ansprüchen 1 bis 3.

5. Stabile, wäßrige Xanthophyll-Dispersionen nach Anspruch 4, **dadurch gekennzeichnet, daß** der enthaltene Wirkstoff einen amorphen Anteil zwischen 70 und 100% aufweist.

6. Stabile, wäßrige Xanthophyll-Dispersionen nach den Ansprüchen 4 und 5, **dadurch gekennzeichnet, daß** der enthaltene Wirkstoff einen all-trans Isomerengehalt von mindestens 50% aufweist.

7. Stabile, wasserdispergierbare Xanthophyll-Trockenpulver **dadurch gekennzeichnet, daß** sie von einem Schutzkolloid umhüllt sind, das mindestens ein niedermolekulares nicht gelierendes Eiweißhydrolysat und mindestens eine höhermolekulare, gelierende Gelatine enthält, deren mittlere Molekulargewichte sich um mindestens 10000 unterscheiden.

8. Verwendung der stabilen, wäßrigen Xanthophyll-Dispersionen und/oder stabilen wasserdispergierbaren Xanthophyll-Trockenpulver nach den Ansprüchen 4 bis 7 als Zusatz zu Lebensmitteln, Pharmazeutika und/oder Tierfuttermitteln.

## Claims

1. A process for preparing a stable aqueous dispersion, or a stable water-dispersible dry powder, of xanthophylls, which comprises
a) preparing a molecularly dispersed solution of at least one xanthophyll, with or without an emulsifier and/or an edible oil, in a water-miscible organic solvent, or a mixture of water and a water-miscible organic solvent, at above 30°C,
b) mixing this solution with an aqueous solution of a mixture of protective colloids,
b₁) in which the mixture comprises at least one low-molecular-weight non-gelling protein hydrolysate and at least one higher-molecular-weight gelling gelatin whose mean molecular weights differ by at least 10,000,
b₂) the solvent component being transferred to the aqueous phase and the hydrophobic phase of the xanthophyll being formed as a nanodisperse phase
c) and if appropriate, to prepare a water-dispersible dry powder, freeing the resulting dispersion from the solvent and the water and drying it in the presence or absence of a coating material.

2. A process as claimed in claim 1, wherein the proportion of the low-molecular-weight non-gelling protein hydrolysate is from 5 to 95 % by weight of the total amount of protective colloid.

3. A process as claimed in claims 1 and 2, wherein the molecularly dispersed solution of the xanthophyll is mixed in two steps with the aqueous protective-colloid-containing phase, the molecularly dispersed solution of the xanthophyll being mixed first of all only with the low-molecular-weight non-gelling protein hydrolysate and then with the higher-molecular-weight gelling gelatin, the mean molecular weights of the low-molecular-weight and higher-molecular-weight polymers differing by at least 30,000.

4. A stable aqueous xanthophyll dispersion obtainable as claimed in claims 1 to 3.

5. A stable aqueous xanthophyll dispersion as claimed in claim 4, wherein the active compound present has an amorphous content from 70 to 100 %.

6. A stable aqueous xanthophyll dispersion as claimed in claims 4 and 5, wherein the active compound present has an all-trans isomer content of at least 50 %.

7. A stable water-dispersible xanthophyll dry powder which is encased by a protective colloid which comprises at least one low-molecular-weight non-gelling protein hydrolysate and at least one higher-molecular-weight gelling gelating whose mean molecular weights differ by at least 10,000.

8. The use of the stable aqueous xanthophyll dispersions and/or stable water-dispersible xanthophyll dry powders as claimed in claims 4 to 7 as an additive to foods, pharmaceuticals and/or animal feeds.

## Revendications

1. Procédé pour la préparation d'une dispersion aqueuse stable ou d'une poudre sèche stable, dispersable dans l'eau de xanthophylles, **caractérisé par le fait qu'**on
a) prépare une solution avec dispersion moléculaire d'au moins un xanthophylle éventuellement conjointement avec un émulsifiant et/ou une huile comestible dans un solvant organique, miscible avec l'eau ou un mélange d'eau et d'un solvant organique, miscible avec l'eau, à des températures supérieures à 30°C,
b) mélange cette solution avec une solution aqueuse d'un mélange de colloïdes protecteurs,
b₁) dans laquelle le mélange contient au moins un hydrolysat d'ovalbumine non gélifiant, de faible masse moléculaire, et au moins une gélatine gélifiante, de masse moléculaire élevée, dont les masses moléculaires moyennes diffèrent d'au moins 10000,
b₂) tandis que le composant solvant est transféré dans la phase aqueuse et la phase hydrophobe du xanthophylle forme une phase nanodispersée,
c) et éventuellement on débarrasse la dispersion formée du solvant et de l'eau pour la préparation d'une poudre sèche dispersable dans l'eau et on sèche, éventuellement en présence d'une matière d'enduction.

2. Procédé selon la revendication 1, **caractérisé par le fait que** la proportion de l'hydrolysat d'ovalbumine non gélifiante, de faible masse moléculaire est de 5 à 95 % en poids de la quantité totale de colloïde protecteur.

3. Procédé selon les revendications 1 et 2, **caractérisé par le fait que** la solution en dispersion moléculaire du xanthophylle est mélangée avec la phase aqueuse, contenant le colloïde protecteur en deux étapes, la solution en dispersion moléculaire du xanthophylle étant mélangée d'abord seulement avec l'hydrolysat d'ovalbumine non gélifiant, de faible masse moléculaire, et ensuite avec la gélatine gélifiante, de masse moléculaire élevée, tandis que les masses moléculaires moyennes des polymères de faible masse moléculaire et de masse moléculaire élevée diffèrent d'au moins 30000.

4. Dispersions aqueuses, stables de xanthophylles pouvant être obtenues selon les revendications 1 à 3.

5. Dispersions aqueuses, stables de xanthophylles selon la revendication 4, **caractérisées par le fait que** la substance active contenue présente une fraction amorphe entre 70 et 100%.

6. Dispersions aqueuses, stables de xanthophylles selon les revendications 4 et 5, **caractérisées par le fait que** la substance active contenue présente une teneur en isomère tout-trans d'au moins 50%.

7. Poudres sèche des xanthophylle stables, dispersables dans l'eau, **caractérisées par le fait qu'**elles sont enrobées d'un colloïde protecteur, qui contient au moins un hydrolysat d'ovalbumine non gélifiant, de faible masse moléculaire et au moins une gélatine gélifiante, de masse moléculaire élevée, dont les masses moléculaires moyennes diffèrent d'au moins 10000.

8. Utilisation des dispersions de xanthophylle aqueuses, stables et/ou des poudres sèches de xanthophylle stables, dispersables dans l'eau selon les revendications 4 à 7, comme additif à des produits alimentaires, des produits pharmaceutiques et/ou des aliments pour animaux.
